# EUROPÄISCHE PATENTSCHRIFT

(11) **EP 0 836 847 B1**
(45) Veröffentlichungstag und Bekanntmachung des Hinweises auf die Patenterteilung: **20.08.2003**
(21) Anmeldenummer: 97250265.2
(22) Anmeldetag: 11.09.1997
(51) Int. Cl.: A61K 7/00

(54) **Stabile Mehrphasenemulsion vom Typ O1/W/O2**
Stable O1/W/O2 multiple emulsion
Emulsion multiple stable de type O1/W/O2

(30) Priorität: 13.09.1996 DE 19638729
(43) Veröffentlichungstag der Anmeldung: 22.04.1998
(73) Patentinhaber: LANCASTER GROUP GmbH, 55116 Mainz (DE)
(72) Erfinder: Ferrero, Louis, 06000 Nice (FR); Golz, Karin, 98000 Monaco (MC); Zastrow, Leonhard, 98000 Monaco (MC); Stanzl, Klaus, White Plains, N.Y. 10605 (US)
(74) Vertreter: Walter, Wolf-Jürgen

(56) Entgegenhaltungen:
- EP-A- 0 281 394
- EP-A- 0 559 013
- US-A- 5 304 334

## Beschreibung

Die Erfindung betrifft eine stabile Mehrphasenemulsion vom Typ O₁/W/O₂ mit hohen Anteilen der inneren Ölphase O₁.

Es sind bereits verschiedene O/W/O-Emulsionen bekannt geworden (O/W/O = Öl-in-Wasser-in-Öl), bei denen die primäre O/W-Emulsion (O/W = Öl-in-Wasser) mit Hilfe hydrophiler ionischer oder nichtionischer Emulgatoren stabil gehalten werden soll (siehe z. B. EP 391124, EP 425958). In der EP 281394 wird mit wenigstens 1,25 % hydrophilen Emulgator Natriumalkylpolyethersulfonat nur 4,5 % Ölphase in der primären Emulsion emulgiert.

Aus der JP-A-07-101849 ist eine Mehrphasenemulsion vom Typ O/W/O bekannt, bei der Kojisäure (5-Hydroxy-2-hydroxymethyl-4H-pyran-4-on) als aktiver Bestandteil enthalten ist. Die JP-A-07-101844 beschreibt eine O/W/O-Emulsion, die zwei verschiedene UV-Filter enthält und eine verringerte Hautreizung aufweist.

Problematisch bei den bekannten Emulsionen dieses Typs ist es, daß insbesondere bei Sonnenschutzzubereitungen mit höheren Lichtschutzfaktoren große Mengen an lipophilen UV-Absorptionsmitteln in die innere Ölphase eingebracht werden müssen und dies bisher nur über die Erhöhung des Anteiles des hydrophilen Emulgators möglich ist. Damit wird jedoch die Stabilität der gesamten Emulsion und somit auch der äußeren Ölphase beeinträchtigt, da herkömmliche O/W/O-Emulsionen dazu neigen, zu einfachen O/W-Emulsionen zu zerfallen.

Weiterhin bekannt ist, daß erhöhte Mengen an Öl, insbesondere an Siliconölen, zu Schwierigkeiten bei der Herstellung stabiler W/O-Formulierungen führten. Es zeigt sich eine schlechte Stabilität, und daraus resultierte eine zu hohe Fluidität. Es besteht daher auch das Bedürfnis, mehr Öl ohne Stabilitätsverlust in die Ölphase einer W/O-Formulierung aufzunehmen.

Der Erfindung liegt die Aufgabe zugrunde, ein neues Mehrphasenemulsionssystem vom Typ O/W/O zu entwickeln, das hohe Anteile der inneren Ölphase enthalten kann.

Ein weiteres Ziel der Erfindung besteht darin, die Hautreizung durch UV-Absorptionsmittel nahezu vollständig zu verhindern.

Ein weiteres Ziel der Erfindung besteht in einem Herstellungsverfahren für Emulsionen vom Typ O/W/O mit hoher Langzeitstabilität.

Erfindungsgemäß besteht die stabile Mehrphasenemulsion vom Typ O₁/W/O₂ aus einer primären Öl-in-Wasser-Phase ohne Emulgator, bei der die inneren Öltröpfchen in einem viskoplastischen wäßrigen Gel im wesentlichen koaleszensfrei in feiner Verteilung zusammen mit einem Gelbildner, vorzugsweise einem amphiphilen Polyacrylat-Block copolymeren vorliegen, wobei der Fließpunkt des viskoplastischen Gels im Bereich von 20 bis 100 Pa liegt und deren plastische Viskosität von 0,01 bis 0,1 Pa·s beträgt, und wobei die innere Ölphase einen Anteil von 10 bis 35 Gewichts-%, bezogen auf die Gesamtemulsion einnimmt und das sekundäre Öl wenigstens einen lipophilen Wirkstoff enthält; und einer sekundären Öl-Phase, in der die primäre O/W-Phase zusammen mit einem lipophilen Emulgator vorliegt.

Zur Erläuterung ist darauf zu verweisen, daß sich die inneren Öltröpfchen (innere Ölphase) stets in der primären O₁/W-Phase befinden.

Gemäß Ross, S. und Morrisson, I. "Colloidal Systems and Interfaces", John Wiley & Sons, New York, S. 17-19, wird das plastische Fließen in Abhängigkeit von Schergeschwindigkeit und Schubspannung ermittelt. Danach beschreibt ein Rheogramm eines plastischen Materials ein Material, das nicht fließt, bevor nicht eine ausreichende Schubspannung ausgeübt wird. Die Schubspannung, bei der das Fließen beginnt, wird als Fließpunkt (yield point) oder Fleißwert bezeichnet. Bei Schubspannungen höher als der Fließpunkt ist die Fließgeschwindigkeit zur Schubspannung nahezu linear.

Die plastische Viskosität ist abgeleitet von der Steigung dieser Kurve im Rheogramm und ist über den experimentell gemessenen Fließpunkt zu errechnen (s.a. Couarraze, G. und Grossiord, J.L. "Initiation a la Rheology", Lavoisier-Tec&Doc, Paris, 2. Aufl. S. 75-78).

Fließpunkte plastischer Körper (die auch als Binghamoder Casson-Körper bezeichnet werden), sind beispielsweise für Sprühlacke ein niedriger Fließpunkt von 0 bis 1 Pa (niedrige plastische Viskosität von 0,01-0,1 Pa·s), für Farben und Ketchup ein mittlerer Fließpunkt von 5-50 Pa (niedrige plastische Viskosität von 0,1 bis 0,5 Pa·s) und für Salben ein hoher Fließpunkt von 100 bis 1000 Pa (mittlere bis hohe plastische Viskosität von 0,5-5 Pa·s).

Für das erfindungsgemäße viskoplastische Gel, d.h. die primäre O₁/W-Emulsion, werden mittlere bis hohe Fließpunkte von 20 bis 100 Pa gemessen bei einer sehr niedrigen plastischen Viskosität von etwa 0,01 Pa·s, wobei diese Werte nach der bekannten Casson-Gleichung bestimmt werden. Die Gelstruktur wird auch nach einem vorübergehenden Abbau der Struktur durch eine hohe Schergeschwindigkeit sehr schnell wieder aufgebaut, und diese auch als Thixotropie bezeichnete Eigenschaft ist wesentlich für das Erfindungskonzept.

Als Gelierungsmittel für die primäre O₁/W-Phase sind alle Gelierungsmittel geeignet, die der wäßrigen Phase ein stark viskoplastisches Verhalten verleihen und die ausreichend stabilisierende und dispergierende Eigenschaften haben, um entsprechend kleine Tröpfchen zu erzeugen, die in der zweiten Stufe von der sekundären Ölphase in feinverteilter Form aufgenommen werden.

Besonders geeignet dafür sind Polyacrylat-Blockcolymere mit alternierenden hydrophilen und hydrophoben Blöcken, insbesondere Hypan-Hydrogele vom TN-Typ. Diese Hydrogele bilden ein feines dreidimensionales Netzwerk im wäßrigen Medium, das durch hohe Scherkräfte und/oder Wärme reversibel abgebaut werden kann. Geeignete Hydrogele sind (CTFA-Namen): Acrylic acid/Acrylonitrogens copolymer wie Hypan SA-100H oder Hypan SR-150H; Ammonium acrylates/Acrylonitrogens copolymer wie Hypan SS-201; Polyquaternium-31 wie Hypan QT-100 (alle hergestellt von Kingston Technology Inc., N. Y., USA).

Das amphiphile Polyacrylat-Blockcopolymere ist dabei verantwortlich für sowohl das viskoplastische Verhalten als auch für die gute Verteilung der inneren Ölphase in dem wäßrigen Gel.

Die Zugabe der Hypan TN-Gele erfolgt in sehr geringen Mengen, die bei 0,2 bis 0,5 Gewichts-%, bezogen auf die Gesamtmasse der Emulsion liegen.

Als Gelbildner kann auch ein Dreischichttonmineral wie Smektit eingesetzt werden. Geeignet sind vor allem synthetische Smektite mit trioktaedrisch koordinierten Kationen, die aus Magnesiumsilicaten und Alkalikationen hergestellt werden, beispielsweise Smectite SWN® (von Nikko Chemicals Corp.). Die Einsatzmengen von Smektiten liegen höher als bei den Hypan-Hydrogelen, d.h. etwa bei 1 - 3 Gew-%.

Man erhält mit Hilfe des Gelbildners ein thixotropes wäßriges Gel mit darin fein verteilten Öltröpfchen, wobei diese primäre Öl-in-Wasser-Phase einen Fließpunkt im Bereich von 20 bis 100 Pa hat. Der Fließpunkt läßt sich weiter erhöhen durch Zugabe von Polyethylenglycol, z. B. durch Zugabe von Glycereth-26 oder einem Polyethylenglycol wie PEG-8.

Weiterhin erfindungswesentlich ist die sehr geringe plastische Viskosität von 0,01 bis 0,1 Pa·s.

Das für die primäre Ölphase eingesetzte Öl ist ein übliches Triglycerid, wie pflanzliche oder synthetische Öle, die üblicherweise in kosmetischen Formulierungen eingesetzt werden. Dazu gehören auch lineare oder verzweigte Ester von Fettsäuren und Alkoholen, Ester von Fettsäuren und Glycolen wie Propylglycolester sowie Ester von Hydroxyfettsäuren. Wesentlich für das primäre Öl ist die Kompatibilität mit den Wirkstoffen der Ölphase und das Zusammenwirken mit den Polyacrylat-Blockcopolymeren, um eine ausreichend dünne (niedrigviskose) primäre O/W-Emulsion auszubilden, die jedoch einen ausreichenden Fließpunkt hat.

Als besonders vorteilhaft für Sonnenschutzpräparate haben sich Ester von Fettsäuren und Alkoholen mit mittleren Kettenlängen erwiesen, wie C12-C13-Alkyloctanoatester, die zusammen mit organischen Sonnenschutzmitteln eingesetzt werden. Andere spezielle Ester wie C12-C13-alkyl Malate, C12-C13-alkyl Lactate und C12-C13-alkyl Citrate können dazugegeben werden.

Unter den pflanzlichen Ölen sind Avocadoöl, Reiskleieöl (rice bran oil), Jojobaöl, Babassuöl bevorzugt.

Es können auch andere Ester eingesetzt werden, wie Diethylenglycoldioctanoat oder -diisononanoat, Propylenglycoldicaprylat, Neopentylglycoldiheptanoat usw.

Die primäre O/W-Emulsion enthält in den primären Öltröpfchen wenigstens einen lipophilen Wirkstoff. Dieser Wirkstoff ist vorzugsweise ein UV-Schutzmittel oder ein UV-Blocker, wie Octyl Methoxycinnamate, Octyl Salicylate; Homosalate; Menthyl Antranilate; Octocrylene; Benzophenone-3; Octyl Dimethyl PABA; 4-Methylbenzilidene Camphor; Butyl Methoxy-Dibenzoylmethane.

Weiterhin können in der primären (inneren) Ölphase und auch in der sekundären (äußeren) Ölphase enthalten sein: fettlösliche Vitamine, wie Vitamin A-Ester (Retinol Palmitate, Acetate); Vitamin E wie Tocopherolacetat oder Tocopherollinolat; Vitamin B2, Vitamin D6; Vitamin F.

Entzündungswidrige Mittel wie Bisabolol, Glycerrethinsäure (Glycerrethinic acid), Stearylglycerrethinat (Stearyl Glycerrhetinate);
Polyungesättigte Fettsäuren oder Fettsäureester davon, wie Öle von Avocado, Erdnuß, Borretsch; Jojobaöl und Calendulaöl usw.;
Unverseifbare, wie Sheabutter, Avocado, Sojabohnen usw.;
Lanolin und Lanolinderivate;
Erweichungsmittel (emollients) wie Perhydrosqualene, Perfluoropolyether.

In der wäßrigen Phase können ebenfalls bestimmte hydrophile Wirkbestandteile enthalten sein, wie Feuchthaltemittel wie z. B. Glycerol, Propylenglycol, verschiedene PEG-Qualitäten, sorbitol, Glucose, Maltose usw.;
Panthenol oder Allantoin;
Peptide oder Proteine und deren Derivate, wie Collagen, Elastin usw.;
Wasserlösliche Vitamine, wie Ascorbinsäure;
Schutzmittel, wie Chlorhexidin, Phenoxyethanol, Dimethylhydantoin, Imidazolidinylharnstoff.

Die sekundäre oder äußere Ölphase, in der die primäre Ölin-Wasser-Emulsion als fein verteilte Tröpfchen emulgiert ist, wird durch ein beliebiges Öl für kosmetische Formulierungen gebildet, wie z. B. Siliconöl, synthetische Fettsäureester, Paraffinöle, Wachse wie Mikrowachse, Bienenwachs, Castorwachs oder Polyethylenwachs.

Als besonders vorteilhaft hat sich Siliconöl erwiesen, insbesondere dann, wenn ein Sonnenschutzpräparat hergestellt werden soll. Siliconöl ist deshalb bevorzugt, weil es sich nicht fettig anfühlt, eine gute Spreitbarkeit auf der Haut zeigt und eine gute Wasserabweisung. Eingesetzt werden können lineare oder cyclische Polydimethylsiloxane, wie Cyclometicone, denen weitere Organopolysiloxane zugesetzt werden können, wie Alkyldimethicone, Alkoxydimethicone (Abil®-Wachse) oder Phenyldimethicone bzw. Phenyltrimethicone.

Den Siliconölen können auch andere geeignete Polymere zugesetzt werden, um die Sperrwirkung und die Fähigkeit zur Filmbildung zu verbessern, wie Siliconacrylate und Vinylsilicone oder Fluorosilicone oder Perfluorpolyether (wie Fomblin®) und auch Silcon-Gums wie Dow Corning 1401® und 1403®.

Für die sekundäre Ölphase können entsprechend den eingesetzten Ölen dafür geeignete Emulgatoren verwendet werden. Wenn Siliconöle die sekundäre Ölphase bilden, sind solche Emulgatoren geeignet, wie Polysiloxan-Polycetyl-Polyethylenglycol-Copolymere (CTFA-Name: Cetyl Dimethicone Copolyol), z. B. Abil® EM90 (Goldschmidt), Abil® WE09 (Goldschmidt), Q2-5200 (Dow Corning). Vorteilhafte sollte der HLB-Wert dieser Emulgatoren nicht größer als 8 sein.

In der sekundären Ölphase können weitere Wirkstoffe enthalten sein. Insbesondere im Falle von Sonnenschutzpräparaten können Sonnenschutzmittel enthalten sein, vorzugsweise anorganische Mineralpigmente wie mikronisiertes TiO₂ oder ZnO. Es können auch andere pulverförmige Produkte, wie Polyamid-12 (Orgasol® von Atochem), Polymethylmethacrylat (Covabead® von Wacker) oder Polymethylsilsesquioxan (Tospearl® von Kobo) zur Verbesserung des Hautgefühls vorhanden sein.

Ein für Sonnenschutzpräparate besonders vorteilhaftes Konzept besteht darin, organische Sonnenschutzmittel, die Irritationswirkungen auf die Haut ausüben können, in der primären Ölphase zu halten, und die hautverträglichen anorganischen Pigmente in der sekundären Ölphase zu dispergieren. Damit erhält man ein besonders hautfreundliches Präparat, das wegen des hohen Anteils an primärer Öl-in-Wasser-Phase und der Möglichkeit, in der primären Ölphase hohe Anteile an organischen Sonnenschutzmittel unterzubringen, sehr hohe Lichtschutzfaktoren erreicht und damit vergleichbaren Präparaten deutlich überlegen ist.

In der erfindungsgemäßen Zubereitung auf Basis einer Mehrphasenemulsion können weitere, bisher nicht benannte Wirkstoffe, Additive und Hilfsstoffe enthalten sein, die in kosmetischen und dermatologischen Zubereitungen üblicherweise eingesetzt werden. Einschränkungen bestehen nur in sofern, daß diese Bestandteile den Aufbau der Mehrphasenemulsion nicht stören dürfen.

In einer bevorzugten Ausführungsform ist in der inneren Ölphase ein mikronisiertes mineralisches Pigment vorhanden, wie z.B. ZnO, TiO₂, SiO₂, ZrO₂, allein oder im Gemisch mit chemischen Filtern. Die Hauptvorteile eines solchen Sonnenschutzpräparates sind (1) ein sehr gutes Hautgefühl (feeling) während des Auftragens der Emulsion auf die Haut, da nur die sekundäre (Silicon)Öl-Phase mit der Haut in Kontakt kommt; (2) eine bessere Wirksamkeit als UV-Schutz als bei organischen Estern, wenn z.B. TiO₂ in der inneren Ölphase vorhanden ist, und die sekundäre Ölphase (z.B. von Siliconöl gebildet) ist gegen Beeinträchtigungen geschützt; und (3) Abschwächung des Weißeln-Effektes, der normalerweise mit physikalischen UV-Absorptionsmitteln verbunden ist, wenn diese mit 5 Gew-% oder mehr in Sonnenschutzpräparaten enthalten sind. Der Bereich an mineralischen Pigmenten in einer Mehrfachemulsion liegt zwischen 0,5 und 8 Gew-%, bezogen auf die Gesamtmasse der Emulsion.

Ebenso sollte die viskoplastische Eigenschaft der primären O₁/W-Emulsion nicht gestört werden z. B. durch Elektrolyte, die das Gelnetzwerk zerstören. Auch der Einsatz von hydrophilem Emulgator mit HLB-Werten von größer als 12 für die äußere Ölphase würde zur Destabilisierung der O₁/W/O₂-Emulsion führen und ist somit nicht zweckmäßig.

Die Erfindung betrifft auch ein Herstellungsverfahren für eine stabile Mehrphasenemulsion vom Typ O₁/W/O₂. Das Verfahren besteht darin, daß folgende Schritte durchgeführt werden:
ein Gelbildner, wie ein Polyacrylat-Blockcopolymeres, wird in einer wäßrigen Phase bis zur Bildung eines Gels dispergiert;
die wäßrige Phase und davon getrennt eine primäre Ölphase werden erwärmt auf eine Temperatur bis maximal 70 °C;
die primäre Ölphase wird durch Anwendung intensiver Scherkräfte bei der erhöhten Temperatur in der wäßrigen Phase dispergiert;
die primäre O/W-Emulsion wird danach unter mäßigem Rühren abgekühlt bis wieder eine Gelbildung auftritt;
die primäre O/W-Emulsion wird in der sekundären Ölphase bei Umgebungstemperatur unter mäßigem Rühren dispergiert.

Gegenstand der Erfindung ist auch die Verwendung einer stabilen Mehrphasenemulsion vom Typ O₁/W/O₂ als Sonnenschutzpräparat oder Make-up-Formulierung mit einer primären Öl-in-Wasser-Phase, bestehend aus einem viskoplastischen Gel, das in den Öltröpfchen wenigstens einen organischen UV-Absorber enthält, und einer sekundären Ölphase aus einem Siliconöl, das einen oder mehrere anorganische UV-Absorber enthält, wobei der Ölanteil in jeder Phase im Bereich von 15 bis 30 Gewichts-% liegt, bezogen auf das Gesamtgewicht der Emulsion.

Die Erfindung soll durch Beispiele näher erläutert werden. Alle Angaben beziehen sich auf das Gewicht, sofern nichts anderes angegeben ist.

### Beispiel 1 Herstellung der Mehrfachemulsion vom Typ O₁/W/O₂

Ein Polyacrylat-Block Copolymeres als Gelbildner wurde in einer wäßrigen Phase dispergiert und ein Gel gebildet. Der pH-Wert wurde auf etwa 6 eingestellt. Zugleich wurde ein Öl auf etwa 70 °C erwärmt und in der Gelphase, die etwa die gleiche Temperatur hatte unter starkem Rühren bei 70 °C für etwa 2 bis 5 Minuten dispergiert; Fließpunkt 54,85 Pa. Der Fließpunkt wurde mittels eines Rheolab MC100 (von Paar Physica) gemessen; Konuswinkel 2°, Abflachung 0,05 mm, Konusdurchmesser 50 mm. Die Auswertungssoftware enthielt eine Berechnung nach der Casson-Methode.

Die Emulsion wurden unter mäßigem Rühren bis zum Eintritt der erneuten Gelbildung gerührt. Danach wurde die sekundäre Ölphase mit einem hydrophoben (lipophilen) Emulgator versetzt und die primäre O/W-Phase unter mäßigem Rühren für 2 bis 5 Minuten bei Raumtemperatur in der sekundären Ölphase dispergiert.

Die Emulsion hatte die folgende Zusammensetzung

| Primäre Emulsion | |
|---|---|
| Wasser | q.s. |
| PEG-8 | 4,0 |
| Polyquaternium-31 | 0,5 |
| C12-C13 Alkyl Octanoate | 21,0 |
| Octyl Methoxycinnamate | 2,0 |
| Aminomethylpropanol (1 %ig) | 1,0 |
| DMDM Hydantoin | 0,5 |

| Sekundäre Ölphase | |
|---|---|
| Cyclometicone | 19,5 |
| Cetyl Dimethicone | 1,5 |
| Cetyl Dimethicone Copolyol | 2,5 |

Die Ausbeute für das eingeschlossene Material bei der Mehrphasenemulsion vom Typ O₁/W/O₂ betrug nach spektroskopischer Messung 81 %, und die Tröpfchen waren in der Emulsion gut erkennbar.

### Beispiel 2 bis 5

Es wurde wie im Beispiel 1 verfahren, jedoch anstelle von C12-C13 Alkyl Octanoate in der primären Emulsion eingesetzt: Sesamöl (Beispiel 2), Isopropylpalmitat (Beispiel 3), Octyldodecylmyristat (Beispiel 4) und Diisopropyl Dimer Dilinoleate (Beispiel 5). Es wurden nahezu gleiche Werten für den Fließpunkt und die Ausbeute wie im Beispiel 1 erhalten.

### Beispiel 6 und 7 Herstellung einer O₁/W/O₂-Sonnenschutzemulsion

Nach der prinzipiellen Arbeitsweise gemäß Beispiel 1 wurden zusätzlich in die primäre O/W-Emulsion zwei organische UV-B-Schutzmittel aufgenommen.

| Primäre Emulsion | Beispiel 6 | Beispiel 7 |
|---|---|---|
| Wasser | q.s. | q.s. |
| Glycereth-26 | 4 | 4 |
| Acrylic acid/ Acrylonitrogen copolymer | 0,5 | 0 |
| Polyquaternium-31 | 0 | 0,5 |
| C12-C13 Alkyl Octanoate | 12 | 12 |
| Tocopherylacetat | 1,0 | 1,0 |
| Octylmethoxycinnamate | 7 | 7 |
| Benzophenone-3 | 3 | 3 |
| Amp95 Sol 1 % | 0 | 2,0 |
| Amp95 Sol 10 % | 2,0 | 0 |
| DMDN Hydantoin | 0,30 | 0,30 |

| Sekundäre Ölphase | | |
|---|---|---|
| Cyclomethicone | 17,0 | 18,0 |
| Cetyldimethicone | 1,0 | 0,5 |
| Cetyl Dimethicone Copolyol | 2 | 2 |

Die Ausbeute der Mehrphasenemulsion vom Typ O₁/W/O₂ beim Beispiel 7 betrug 88 %, wobei das Aussehen der Emulsion gelartig und sehr gut war (Fließpunkt 55,9 Pa). Die Emulsion von Beispiel 6 hatte eine eher flüssige Konsistenz (Fließpunkt 21,7 Pa).

Mit insgesamt 10 % war der Anteil der Sonnenschutzmittel in der primären Emulsion sehr hoch und lag weit über den bisher gekannten Werten.

Die Stabilität der Emulsion von Beispiel 7 war ebenfalls sehr gut, sie lag sowohl bei Temperaturen von 4 °C als auch bei 40° C und 50° C bei einem Wert von über 6 Monate.

Der Sonnenschutzfaktor (SPF) bei der Emulsion von Beispiel 7 lag bei 16 (Standardabweichung S.D.= 2,81).

### Beispiel 8 und 9 Herstellung von O₁/W/O₂-Sonnenschutzemulsionen

Nach der prinzipiellen Arbeitsweise gemäß Beispiel 1 und 6 sowie 7 wurde zusätzlich in die primäre O/W-Emulsion ein drittes Sonnenschutzmittel aufgenommen (Beispiel 8) bzw. in die sekundäre Ölphase ein mineralisches UV-Absorptionsmittel (Beispiel 9).

| Primäre Emulsion | Beispiel 8 | Beispiel 9 |
|---|---|---|
| Wasser | q.s. | q.s. |
| Glycereth-26 | 4 | 4 |
| Polyquaternium-31 | 1,0 | 0,5 |
| C12-C13 Alkyl Octanoate | 10,0 | 12,0 |
| Tocopherylacetate | 1,0 | 0,5 |
| Octylmethoxycinnamate | 7 | 7 |
| Benzophenone-3 | 3 | 3 |
| Butyl Methoxy Dibenzoylmethane | 2,0 | 0 |
| Amp95 Sol. 1 % | 1 | 1 |
| DMDM Hydantoin | 0 | 0,5 |
| Phenoxyethanol und PHB | 1,0 | 0 |

| Sekundäre Ölphase | | |
|---|---|---|
| Cyclomethicone | 17,5 | 12 |
| Cetyldimethicone | 0,5 | 0,5 |
| 40% TiO₂ + C12-15 Alcohols Benzoate | 0 | 5 |
| Cetyl Dimethicone Copolyol | 2 | 2,5 |

Man erhielt beide Emulsionen mit einem sehr guten gelartigen Aussehen, wobei die Ausbeute an Mehrfachemulsion des Beispiels 8 etwa 87 % betrug.

Für die Emulsion von Beispiel 8 wurde ein SPF von 19 gemessen (SD=2,03) und für die Emulsion von Beispiel 9 ein SPF von 24,0 (SD=3,10).

Der Aufbau der Emulsion mit dem chemischen Sonnenschutzmittel in der primären Ölphase und dem physikalischen UV-Absorptionsmittel in der sekundären Ölphase ließ sich mikroskopisch in polarisierten Licht deutlich nachweisen. Aus Mikrophotographien ist zu entnehmen, wie mikronisiertes TiO₂ hell in der sekundären Phase vorliegt, und die O/W-Tröpfchen sich dunkel dagegen abheben mit schwarzen Öltröpfchen der primären Ölphase darin.

### Beispiel 10 und 11 O₁/W/O₂-Sonnenschutzemulsion mit aktiven Zusatzstoffen

Nach der prinzipiellen Arbeitsweise von Beispiel 1 wurden zusätzlich zu den UV-Filtern weitere kosmetisch übliche Aktivstoffe, wie Befeuchtungsmittel, entzündungswidrige Mittel, Wundheilungsmittel, Radikalfänger, Antioxidationsmittel sowie pulverförmige Sonnenprodukte in der inneren oder äußeren Ölphase zugegeben.

| | Beispiel 10 | Beispiel 11 |
|---|---|---|
| Primäre Emulsion | | |
| Wasser | q.s. | q.s. |
| Glycereth-26 | 4 | 4 |
| Polyquaternium-31 | 0,5 | 0,5 |
| Glycerol | 7 | 0 |
| DL Panthenol | 2 | 0 |
| Allantoin | 0,5 | 0 |
| Amp.95 Sol. 1 % | 1,0 | 1,0 |
| C12-C13 Alkyl Octanoate | 9,0 | 12,0 |
| Tocopherylacetate | 2 | 1,0 |
| Octylmethoxycinnamate | 7 | 7 |
| Benzophenone-3 | 3 | 3 |
| Stearyl Glycerrethinate | 0,5 | 0 |
| Tocopherol, Ascorbylpalmitat, Ascorbinsäure und Citronensäure | 0,1 | 0 |
| Bisabolol | 0,5 | 0 |
| Parfüm | 0,5 | 0 |
| DMDM Hydantoin | 0,3 | 0,3 |
| Fließpunkt | 47,7 | |

| Sekundäre Ölphase | | |
|---|---|---|
| Cyclomethicone | 18,0 | 17,0 |
| Cetyldimethicone | 0,5 | 0,5 |
| Tospearl 145 | 0 | 1 |
| Cetyl Dimethicone Copolyol | 2 | 2 |

Die Ausbeute der Mehrfachemulsion betrug 89 %. In beiden Fällen wurden stabile, gut aussehende Emulsionen erhalten.

### Beispiel 12 O₁/W/O₂-Mehrphasenemulsion als Hautschutzcreme

Nach der prinzipiellen Arbeitsweise von Beispiel 1 wurden anstelle von UV-Absorptionsmitteln hautpflegende und faltenmindernde Zusatzstoffe zugegeben.

| Primäre Emulsion | |
|---|---|
| Wasser | q.s. |
| PEG-8 | 4 |
| Polyquaternium-31 | 0,5 |
| Glycerol | 5 |
| Amp 95 (1 %ig) | 1,0 |
| C12-C13 Alkyl Octanoate | 15,0 |
| Tocopherylacetate | 2 |
| Rhetinol Acetate 1 Million U/g | 0,2 |
| Babassu-Öl | 3 |
| Octyl Methoxycinnamate | 2 |
| Bisabolol | 1,0 |
| DMDM Hydantoin | 0,3 |

| Sekundäre Ölphase | |
|---|---|
| Cyclomethicone | 18,0 |
| Cetyldimethicone | 0,5 |
| Cetyl Dimethicone Copolyol | 2 |

### Beispiel 13 O/W/O-Mehrphasenemulsion mit erhöhtem Ölanteil

Nach der Arbeitsweise von Beispiel 1 und den dort genannten Bestandteilen wurde die primäre O₁/W-Emulsion mit einem um 10 % höheren Ölanteil gebildet. Ungeachtet der Erhöhung des Gesamtvolumens der Ölphase, blieb die Konsistenz der Mehrfachemulsion hoch und vergleichbar zum Beispiel 1.

Die Emulsion zeigte gute Stabilität und eine gute gel-artige Konsistenz und ihre Eigenschaften entsprachen etwa denen von Beispiel 1.

Auch im umgekehrten Fall, d.h. Erhöhung des Ölanteiles der äußeren Ölphase, ergab sich eine stabile Mehrphasenemulsion. Gegenüber der Erhöhung des Ölanteils in der inneren Ölphase war die gel-artige Konsistenz allerdings nicht so ausgeprägt. Es ist interessanter, den Öl-überschuß bei der primären O₁/W-Emulsion zuzugeben, also in der inneren Ölphase der fertigen Mehrfachemulsion. Auf diese Weise erhält man eine stabilere Mehrfachemulsion mit gel-artiger Konsistenz.

### Vergleichsbeispiel 1

Es wurde eine W/O-Emulsion nach dem Stand der Technik hergestellt.

| | |
|---|---|
| Wasser | q.s. |
| Natriumchlorid | 0,5 |
| C12-C13 Alkyl Octanoate | 12,0 |
| Tocopherylacetate | 1,0 |
| Octylmethoxycinnamate | 7 |
| Benzophenone-3 | 3 |
| DMDM Hydantoin | 0,3 |
| Cyclomethicone | 18,0 |
| Cetyl Dimeticone | 0,5 |
| Cetyl Dimethicone Copolyol | 2 |
| Gesamt | 100 |

Die konventionelle Emulsion, die eine Zusammensetzung sehr ähnlich wie die der Formel von Beispiel 7, ist sehr flüssig. Die Wassertröpfchen waren sehr klein, jedoch war die Stabilität für kosmetische Zwecke nicht ausreichend. Es erfolgte Phasentrennung bei Raumtemperatur nach 3 Monaten, bei 40 °C nach 1 Monat.

Die Emulsion hatte eine sehr niedrige Viskosität von etwa 268 mPa·s und zeigte im Vergleich zur O₁/W/O₂-Emulsion der Erfindung nach Beispiel 7 mit steigender Schergeschwindigkeit [1/s] nur einen geringen Anstieg der Schubspannung τ [Pa], während dies bei der Emulsion gemäß Beispiel 7 umgekehrt war. Eine gel-artige Struktur war daher mit der Emulsion obiger Zusammensetzung nicht zu erreichen.

### Beispiel 14

Nach der prinzipiellen Arbeitsweise von Beispiel 1 wurde das Siliconöl in der äußeren Ölphase durch einen organischen Ester ersetzt. Anstelle von Cetyl Dimethicone Dipolyol wurde ein anderer lipophiler Emulgator eingesetzt.

| Primäre Emulsion | |
|---|---|
| Wasser | q.s. |
| PEG-8 | 3 |
| Polyquaternium-31 | 0,5 |
| C12-C13 Alkyl Octanoate | 23 |
| Octylmethoxycinnamate | 2,0 |
| Amp95 Sol 1 % | 1,0 |
| DMDN Hydantoin | 0,5 |

| Mehrphasenemulsion | |
|---|---|
| Hexyllaurate | 13 |
| Sorbitan Oleate + Polyglyceryl-3 Ricinoleate | 2 |
| Gesamt | 100 |

Die gebildete langzeitstabile O₁/W/O₂-Emulsion zeigt, daß auch klassische Öle eingesetzt werden können.

### Beispiel 15

Nach der prinzipiellen Arbeitsweise von Beispiel 1 wurde die viskoplastische primäre Emulsion O₁/W mit einem anderen gelbildner hergestellt, und zwar mit einem Smektit, der wasserquellbar ist. Der eingesetzte Smektit war ein synthetischer Hectorit.

| Primäre Emulsion | |
|---|---|
| Wasser | q.s. |
| PEG-8 | 4,0 |
| Smektit (Hectorit) | 1,5 |
| Citronensäure (20 %ig) | 0,5 |
| Diisopropyl Dimer Dilinolate | 12 |
| hydriertes Lecithin | 1,0 |
| Octyl Methoxycinnamate | 7 |
| Benzophenone-3 | 3 |
| DMDM Hydantoin | 0,5 |

| Mehrfachemulsion | |
|---|---|
| Cyclometicone | 18,0 |
| Cetyl Dimethicone | 0,5 |
| Cetyl Dimethicone Copolyol | 2,0 |
| Gesamt | 100 |

Die primäre Emulsion hatte einen Fließpunkt von 44,3 Pa und eine plastische Viskosität von nur 0,0032 Pa·s. Die Tröpfchengröße wurde mit einem Emulsionsmodifikator beeinflußt.

### Beispiel 16

Nach der prinzipiellen Arbeitsweise von Beispiel 1 wurde eine erfindungsgemäße Emulsion mit zusätzlichen Farbpigmenten in der äußeren Phase hergestellt. Diese Emulsion ist für Makeup's verwendbar.

| Primäre Emulsion | |
|---|---|
| Wasser | q.s. |
| PEG-8 | 4,0 |
| Polyquaternium-31 | 0,4 |
| C12-C13 Alkyl Octanoate | 23,0 |
| Tocopherylacetat | 1,0 |
| Amp 95 (1 %ig) | 1,0 |
| DMDM Hydantoin | 0,5 |

| Mehrfachemulsion | |
|---|---|
| Isohexadecan | 2,5 |
| Covasil S weiß | 4 |
| Covasil S gelb | 2,0 |
| Covasil s rot | 0,5 |
| Covasil S schwarz | 0,5 |
| Neopentylglycol Diheptanoat | 7,0 |
| Polyethylen | 2 |
| Sorbitan Oleate + Polyglyceryl-3 Ricinoleate | 2 |
| Gesamt | 100 |

### Beispiel 17

Nach der prinzipiellen Arbeitsweise von Beispiel 1 wurde eine erfindungsgemäße Emulsion mit zusätzlichem mikronisiertem TiO₂ in der inneren Ölphase hergestellt.

| Primäre Emulsion | |
|---|---|
| Wasser | q.s. |
| PEG-8 | 4,0 |
| Polyquaternium-31 | 0,5 |
| Aminomethylpropanol (Sol. 1%) | 1,0 |
| C₁₂-C₁₃ Alkyl Octanoate | 15,0 |
| Tocopheryl Acetate | 0,5 |
| Octalmethocycinnamate | 7,0 |
| Mikronisiertes TiO₂ | 5,0 |
| DMDM Hydantoin | 0,5 |

| Mehrphasenemulsion | |
|---|---|
| | |
| Cyclomethicone | 17,5 |
| Cetyl Dimethicone | 0,5 |
| Cetyl Dimethicone Copolyol | 2,0 |
| | |
| Total | 100 |

## Patentansprüche

1. Stabile Mehrphasenemulsion vom Typ O₁/W/O₂, **gekennzeichnet durch**
(a) eine emulgatorfreie primäre Öl-in-Wasser Phase, bestehend aus einem viskoplastischen wäßrigen Gel, das die fein verteilten inneren Öltröpfchen zusammen mit einem Gelbildner enthält, wobei die thixotrope primäre Öl-in-Wasser-Phase einen Fließpunkt im Bereich von 20 bis 100 Pa hat, eine plastische Viskosität von 0,01 bis 0,1 Pa·s hat und wenigstens einen lipophilen Wirkstoff in dem primären Öl enthält, und wobei der Gelbildner ausgewählt ist unter einem Polyacrylat-Blockcopolymeren und einem in Wasser quellbaren Dreischichttonmineral; und
(b) eine sekundäre Ölphase, in der die primäre Öl-in-Wasser-Phase zusammen mit einem lipophilen Emulgator vorliegt; und wobei
(c) der Anteil der inneren Ölphase 10 bis 35 Gew-%, bezogen auf das Gesamtgewicht der Emulsion, beträgt.

2. Mehrphasenemulsion nach Anspruch 1, **dadurch gekennzeichnet daß** der Gelbildner ein Hypan-TN®-Hydrogel, ein Polyquaternium" oder ein Smektit ist.

3. Mehrphasenemulsion nach Anspruch 1, **dadurch gekennzeichnet daß** das primäre Öl ein Öl umfaßt, das aus der Gruppe ausgewählt ist, die lineare oder verzweigte organische Ester, polyungesättigte Triglyceride oder Ester von Hydroxyfettsäuren, Ester von Glycolen umfaßt.

4. Mehrphasenemulsion nach Anspruch 1, **dadurch gekennzeichnet daß** die primäre Öl-in-Wasser-Phase zur Verstärkung der viskoplastischen Eigenschaften zusätzlich ein Glycereth-Additiv oder Polyethylglycol enthält.

5. Mehrphasenemulsion nach Anspruch 1, **dadurch gekennzeichnet daß** die innere oder die äußere Ölphase weitere aktiv wirksame lipophile Zusatzstoffe enthält, ausgewählt aus der Gruppe, die aus UV-Absorptionsmitteln, Vitaminen, entzündungswidrigen Mitteln, polyungesättigten Fettsäuren oder Fettsäureestern wie Avocadoöl, Erdnußöl, Jojobaöl, Calendulaöl; Unverseifbaren wie Sheabutter, Avocado; Lanolin und Lanolinderivaten; Erweichungsmitteln wie Perhydrosqualene, Perfluorpolyether; und Polymeren, die für die Haut schützende Eigenschaften haben durch Abschwächung der Hautpenetration durch organische UV-Absorptionsmittel;
besteht.

6. Mehrphasenemulsion nach Anspruch 1, **dadurch gekennzeichnet daß** die wäßrige Phase zusätzlich hydrophile Wirkstoffe enthält, ausgewählt aus der Gruppe, die aus Feuchthaltemitteln, entzündungswidrigen Mitteln, Peptiden, Proteinen, wasserlöslichen Vitaminen und Schutzmitteln besteht.

7. Mehrphasenemulsion nach Anspruch 1, **dadurch gekennzeichnet daß** die sekundäre Ölphase ein Öl umfaßt, ausgewählt aus der Gruppe, die aus Siliconöl, synthetische Fettsäureester, Paraffinöle, Wachse besteht.

8. Mehrphasenemulsion nach Anspruch 1, **dadurch gekennzeichnet daß** die sekundäre Ölphase einen Emulgator umfaßt mit einem HLB-Wert von ≤8.

9. Mehrphasenemulsion nach Anspruch 1, **dadurch gekennzeichnet daß** die sekundäre Ölphase zusätzlich aktive Wirkstoffe umfaßt, ausgewählt aus der Gruppe anorganische, mikronisierte UV-Absorptionsmittel, wie TiO₂ und ZnO, und pulverförmige Produkte, wie Polyamid-12, Polymethylmethacrylat und Polymethylsilsesquioxan.

10. Mehrphasenemulsion nach Anspruch 1, **dadurch gekennzeichnet daß** der Anteil der sekundären Ölphase im Bereich von 5 bis 30 Gewichts-%, vorzugsweise 18 - 22 Gew-% liegt.

11. Mehrphasenemulsion nach Anspruch 1, **dadurch gekennzeichnet daß** die primäre Ölphase ein organisches UV-Absorptionsmittel und die sekundäre Ölphase ein anorganisches mineralisches UV-Absorptionsmittel wie TiO₂ oder ZnO enthält.

12. Mehrphasenemulsion nach Anspruch 1, **dadurch gekennzeichnet daß** die primäre und die sekundäre Ölphase erforderlichenfalls zusammen einen Anteil von mehr als 50 % einnehmen, bezogen auf die Gesamtemulsion, wobei die leichten Öle in der sekundären Phase enthalten sind.

13. Mehrphasenemulsion nach Anspruch 1, **dadurch gekennzeichnet daß** in der inneren Ölphase ein mikronisiertes mineralisches Pigment, wie TiO₂, ZnO, SiO₂, ZrO₂ in einer Menge von 0,5 bis 8 Gew-% enthalten ist.

14. Verfahren zur Herstellung einer stabilen Mehrphasenemulsion vom Typ O/W/O, **gekennzeichnet durch** folgende Schritte:
ein Gelbildner, wie ein Polyacrylat-Blockcopolymeres, wird in einer wäßrigen Phase bis zur Bildung eines Gels dispergiert;
die wäßrige Phase und separat davon eine primäre Ölphase werden erwärmt auf eine Temperatur bis 65 °C;
die primäre Ölphase wird **durch** Anwendung intensiver Scherkräfte bei der erhöhten Temperatur bis 70 °C in der wäßrigen Phase dispergiert;
die primäre O/W-Emulsion wird danach unter mäßigem Rühren abgekühlt bis wieder eine Gelbildung auftritt;
die primäre O/W-Emulsion wird in der sekundären Ölphase bei Umgebungstemperatur unter mäßigem Rühren dispergiert.

15. Verwendung einer stabilen Mehrphasenemulsion vom Typ O₁/W/O₂ mit einer primären Öl-in-Wasser-Phase, bestehend aus einem viskoplastischen Gel, das in den Öltröpfchen wenigstens einen organischen UV-Absorber enthält, und einer sekundären Ölphase aus einem Siliconöl, das einen oder mehrere anorganische UV-Absorber enthält, wobei der Anteil der primären Ölphase im Bereich von 10 bis 35 Gew-% und der Anteil der sekundären Ölphase im Bereich von 15 bis 30 Gew-% liegt, jeweils bezogen auf das Gesamtgewicht der Emulsion, als Sonnenschutzpräparat.

16. Verwendung nach Anspruch 15 als Make-up, wobei die äußere Ölphase Zusätze von Farbpigmenten enthält.

## Claims

1. Stable multiple phase emulsion of the type O₁/W/O₂, **characterized by**
(a) a primary oil-in-water phase without emulsifier comprising a viscoplastic aqueous gel containing finely distributed oil droplets together with a gelling agent, wherein the thixotropic primary oil-in-water phase has a yield point in the range from 20 to 100 Pa, a plastic viscosity from 0.01 to 0.1 Pa·s and contains at least one lipophilic agent in the primary oil and wherein the gelling agent is chosen among a polyacrylate block copolymer and a three-layer clay mineral capable of water swelling; and
(b) a secondary oil phase in which the primary oil-in-water phase is present together with a lipophilic emulsifier; and wherein
(c) the proportion of the inner oily phase is 10 to 35 % by weight relative to the total weight of the emulsion.

2. Multiple phase emulsion according to claim 1, wherein the gelling agent is a Hypan-TN® hydrogel, a polyquaternium or a smectite.

3. Multiple phase emulsion according to claim 1, wherein the primary oil includes an oil selected from the group consisting of linear or branched organic esters, polyunsaturated triglycerides or esters of hydroxy fatty acids and esters of glycols.

4. Multiple phase emulsion according to claim 1, wherein the primary oil-in-water phase for amplification of the viscoplastic characteristics additionally contains a Glycereth additive or a polyethylene glycol.

5. Multiple phase emulsion according to claim 1, wherein the inner or outer oil phase contains further actively effective lipophilic additives selected from the group consisting of UV-absorbents, vitamins, anti-inflammatory agents, polyunsaturated fatty acids or fatty acid esters such as avocado oil, peanut oil, jojoba oil, calendula oil; unsaponables such as shea butter, avocado; lanolin and lanolin derivatives; emollients such as perhydrosqualene, perfluoro polyethers; and polymers which have skin protecting characteristics in view of migrationg skin penetration of organic UV-absorbents.

6. Multiple phase emulsion according to claim 1, wherein the aqueous phase additionally contains hydrophilic agents selected from the group consisting of humectants, anti-inflammatory agents, peptides, proteins, water soluble vitamins and preservatives.

7. Multiple phase emulsion according to claim 1, wherein the secondary oil phase comprises an oil selected from the group consisting of silicone oil, synthetic fatty acid esters, paraffin oils and waxes.

8. Multiple phase emulsion according to claim 1, wherein the secondary oil phase includes an emulsifier with an HLB value ≤8.

9. Multiple phase emulsion according to claim 1, wherein the secondary oil phase additionally comprises active agents selected from the group consisting of micronized inorganic absorbents such as TiO₂ and ZnO, powdery products such as Polyamid-12, polymethyl methacrylate and polymethyl silsesquioxane.

10. Multiple phase emulsion according to claim 1, wherein the proportion of the secondary oil phase is in the range from 5 to 30 % by weight, preferably 18-22 % by weight.

11. Multiple phase emulsion according to claim 1, wherein the primary oil phase contains an organic UV-absorbent and the secondary oil phase contains an inorganic mineral UV-absorbent such as TiO₂ or ZnO.

12. Multiple phase emulsion according to claim 1, wherein the primary and the secondary oil phases together have a proportion by weight ≥ 50 % relative to the total emulsion, and wherein the light oils are present in the secondary phase.

13. Multiple phase emulsion according to claim 1, wherein the inner oily phase contains a micronized mineral pigment such as TiO₂, SiO₂, ZnO, ZrO₂ in an amount of 0.5 to 8 % by weight.

14. Process for the production of a stable multiple phase emulsion of the type O/W/O, **characterized by** the following steps:
a gelling agent such as a polyacrylate block copolymer is dispersed in an aqueous phase until the formation of a gel;
the aqueous phase and a primary oil phase are heated separately to a temperature up to 65 °C;
the primary oil phase is dispersed in the aqueous phase by application of intensive shear stress at an increased temperature up to 70 °C;
the primary O/W-emulsion is subsequently cooled under moderate stirring until gel formation occurs again;
the primary O/W-emulsion is dispersed in the secondary oil phase at the surroundings temperature under moderate stirring.

15. Use of a stable multiple phase emulsion of the type O₁/W/O₂ as a sun protection preparation, wherein the emulsion has a primary oil-in-water phase comprising a viscoplastic gel containing at least one organic UV-absorbent in the oil droplets, and a secondary oil phase formed by a silicone oil, that secondary oil phase containing one or more inorganic UV-absorbents, and wherein the proportion of the secondary oily phase is in the range from 15 to 30 % by weight and wherein the proportion of the primary oily phase is in the range from 10 to 35 % by weight, each relative to the total weight of the emulsion.

16. Use according to claim 15 as a make-up wherein the secondary oil phase contains additional color pigments.

## Revendications

1. Emulsion à plusieurs phases stable, de type H₁/E/H₂ **caractérisée par**
(a) une phase huile-dans-eau primaire sans émulsionnant, consistant en un gel aqueux viscoélastique, qui contient les gouttelettes internes d'huile finement divisées avec un agent formant gel, où la phase huile-dans-eau primaire thixotrope a un point de trouble d'écoulement dans l'intervalle allant de 20 à 100 Pa, une viscosité plastique de 0,01 à 0,1 Pa.s et qui contient au moins un agent actif lipophile dans l'huile primaire et où l'agent formant gel est choisi parmi un copolymère séquencé polyacrylate et un minéral d'argile à trois couches, gonflables dans l'eau, et
(b) une phase huileuse secondaire, dans laquelle la phase huile-dans-eau primaire est présente avec un émulsionnant lipophile, et où
(c) la fraction de la phase huileuse interne se situe de 10 à 35% en poids, sur base du poids total de l'émulsion.

2. Emulsion à plusieurs phases selon la revendication 1, **caractérisée en ce que** l'agent formant gel est un hydrogel Hypan-TN®, un polyquaternium ou une smectite.

3. Emulsion à plusieurs phases selon la revendication 1, **caractérisée en ce que** l'huile primaire comprend une huile qui est choisie parmi le groupe qui comprend des esters organiques linéaires ou ramifiés, des triglycérides ou esters polyinsaturés d'acides gras hydroxylés, des esters de glycol.

4. Emulsion à plusieurs phases selon la revendication 1, **caractérisée en ce que** la phase huile-dans-eau primaire contient en outre, un additif glycereth ou un polyéthylglycol pour renforcer les propriétés viscoplastiques.

5. Emulsion à plusieurs phases selon la revendication 1, **caractérisée en ce que** la phase huileuse interne ou externe contient d'autres additifs lipophiles actifs, choisis parmi le groupe qui consiste en les agents d'absorption des U.V., les vitamines, les agents anti-inflammatoires, les acides gras ou esters d'acides gras polyinsaturés, comme l'huile d'avocat, l'huile d'arachide, l'huile de jojoba, l'huile de calendula ; les huiles non saponifiables comme le sheabutter, d'avocat ; la lanoline et des dérivés de la lanoline ; les émollients comme le perhydrosqualène, un perfluoropolyéther, et des polymères qui ont des propriétés protectrices pour la peau par affaiblissement de la pénétration de la peau par les agents organiques d'absorption des U.V.

6. Emulsion à plusieurs phases selon la revendication 1, **caractérisée en ce que** la phase aqueuse contient en outre, des agents actifs hydrophiles, choisis parmi le groupe qui consiste en les agents hydratants, des agents anti-inflammatoires, des peptides, des protéines, des vitamines hydrosolubles et des agents de protection.

7. Emulsion à plusieurs phases selon la revendication 1, **caractérisée en ce que** la phase huileuse secondaire comprend une huile, choisie parmi le groupe qui consiste en une huile de silicone, des esters d'acide gras synthétiques, l'huile de paraffine, des cires.

8. Emulsion à plusieurs phases selon la revendication 1, **caractérisée en ce que** la phase huileuse secondaire comprend un émulsionnant avec une valeur EHL ≤ 8.

9. Emulsion à plusieurs phases selon la revendication 1, **caractérisée en ce que** la phase huileuse secondaire comprend des agents actifs supplémentaires, choisis parmi le groupe des agents d'absorption des U.V. micronisés, inorganiques, comme le TiO₂ et le ZnO, et des produits pulvérulents, comme le polyamide-12, un poly(méthacrylate de méthyle) et un polyméthylsilsesquioxane.

10. Emulsion à plusieurs phases selon la revendication 1, **caractérisée en ce que** la fraction de la phase huileuse secondaire se situe dans l'intervalle allant de 5 à 30% en poids, de préférence de 18 à 22% en poids.

11. Emulsion à plusieurs phases selon la revendication 1, **caractérisée en ce que** la phase huileuse primaire contient un agent organique d'absorption des U.V. et la phase huileuse secondaire, un agent inorganique minéral d'absorption des U.V. comme le TiO₂ ou le ZnO.

12. Emulsion à plusieurs phases selon la revendication 1, **caractérisée en ce que** la phase huileuse primaire et la phase huileuse secondaire occupent ensemble, si nécessaire, une fraction de plus de 50%, sur base de l'émulsion totale, où les huiles plus légères sont présentes dans la phase secondaire.

13. Emulsion à plusieurs phases selon la revendication 1, **caractérisée en ce que** dans la phase huileuse interne, est présent un pigment minéral micronisé, comme TiO₂, ZnO, SiO₂, ZrO₂ en une quantité de 0,5 à 8% en poids.

14. Procédé de préparation d'une émulsion stable à plusieurs phases du type H/E/H, **caractérisé par** les étapes suivantes :
un agent formant gel, comme un copolymère séquencé polyacrylate, est dispersé dans une phase aqueuse jusqu'à la formation d'un gel ;
la phase aqueuse et une phase huileuse primaire séparée de celle-ci sont chauffées à une température de jusqu'à 65°C ;
la phase huileuse primaire est dispersée dans la phase aqueuse par utilisation de forces de cisaillement importantes à la température élevée de jusqu'à 70°C ;
l'émulsion H/E primaire est alors refroidie sous agitation vigoureuse jusqu'à ce qu'il se produise à nouveau la formation d'un gel ;
l'émulsion H/E primaire est dispersée dans la phase huileuse secondaire à la température ambiante sous agitation vigoureuse.

15. Utilisation d'une émulsion stable à plusieurs phases du type H₁/E/H₂, avec une phase huile-dans-eau primaire, consistant en un gel viscoplastique, qui contient au moins un agent absorbant les U.V. dans les gouttelettes huileuses, et une phase huileuse secondaire en une huile de silicone, qui contient un ou plusieurs agents inorganiques absorbant les U.V., où la fraction de la phase huileuse primaire se situe dans l'intervalle allant de 10 à 35% en poids et la fraction de la phase huileuse secondaire se situe dans l'intervalle allant de 15 à 30% en poids, chaque fois sur base du poids total de l'émulsion, comme préparation de protection solaire.

16. Utilisation selon la revendication 15 comme maquillage, où la phase huileuse externe contient en outre, des pigments colorés.
